# EUROPEAN PATENT SPECIFICATION

(11) **EP 2 324 116 B1**
(45) Date of publication and mention of the grant of the patent: **26.12.2012**
(21) Application number: 09802300.5
(22) Date of filing: 13.07.2009
(51) Int. Cl.: C12N 15/53, A01H 1/00, A01H 1/04, C12N 15/63, C12N 9/04, C12P 17/18, C12Q 1/68

(54) **NUCLEOTIDE SEQUENCE ENCODING AN ALCOHOL DEHYDROGENASE FROM ARTEMISIA ANNUA AND USES THEREOF**
NUKLEOTIDSEQUENZKODIERUNG EINER ALKOHOLDEHYDROGENASE AUS ARTEMISIA ANNUA UND IHRE VERWENDUNG
SÉQUENCE NUCLÉOTIDIQUE CODANT UNE ALCOOL DÉSHYDROGÉNASE PROVENANT D'ARTEMISIA ANNUA ET SES UTILISATIONS

(30) Priority: 01.08.2008 US 137701 P
(43) Date of publication of application: 25.05.2011
(73) Proprietor: National Research Council of Canada, Ottawa, ON K1A OR6 (CA)
(72) Inventor: POLICHUK, Devin, Saskatoon Saskatchewan S7H 0L3 (CA); TEOH, Keat (Thomas) H., Jonesboro Arkansas 72401 (US); ZHANG, Yansheng, Moshan, Wuchang Wuhan 430074 (CN); ELLENS, Kenneth W., Gainsville, FL 32607-2044 (US); REED, Darwin W., Saskatoon Saskatchewan S7M 4J9 (CA); COVELLO, Patrick S., Saskatoon Saskatchewan S7V 1 G8 (CA)
(74) Representative: Graf Glück Habersack Kritzenberger
(86) International application number: PCT/CA2009/000966
(87) International publication number: WO 2010/012074

(56) References cited:
- BERTEA C M ET AL: "Identification of intermediates and enzymes involved in the early steps of artemisinin biosynthesis in Artemisia annua", PLANTA MEDICA, THIEME VERLAG, DE, vol. 71, no. 1, 1 January 2005 (2005-01-01), pages 40-47, XP002541677, ISSN: 0032-0943, DOI: 10.1055/S-2005-837749
- COVELLO ET AL: "Functional genomics and the biosynthesis of artemisinin", PHYTOCHEMISTRY, PERGAMON PRESS, GB, vol. 68, no. 14, 1 July 2007 (2007-07-01), pages 1864-1871, XP022145444, ISSN: 0031-9422, DOI: 10.1016/J.PHYTOCHEM.2007.02.016
- BOUWMEESTER H. ET AL.: "Research to improve artemisinin production for use in the preparation of anti-malaria drugs", MEDICINAL AND AROMATIC PLANTS, 1 January 2006 (2006-01-01), - 1 January 2006 (2006-01-01), pages 275-290, XP002662784,
- DATABASE GENBANK [Online] 18 April 2005 VAN ELDIK, G .J. ET AL.: 'An alcohol dehydrogenase-like gene is pistil-specifically expressed in Solanum.', XP008137981 Database accession no. X92179.
- RO, D. K. ET AL.: 'Production of the antimalaria drug precursor artemisinic acid in engineered yeast' NATURE. vol. 440, 13 April 2006, pages 940 - 943, XP002510909

### SEQUENCE LISTING

<110> National Research Council of Canada
   POLICHUK, Devin
   TEOH, Keat (Thomas)
   ZHANG, Yansheng
   ELLENS, Ken
   REED, Darwin W.
   COVELLO, Patrick S.
<120> NUCLEOTIDE SEQUENCE ENCODING AN ALCOHOL DEHYDROGENASE FROM ARTEMISIA ANNUA AND USES THEREOF
<130> 12062-98
<150> 61/137,701 <151> 2008-08-01
<160> 2
<170> PatentIn version 3.5
<210> 1
   <211> 1137
   <212> DNA
   <213> Artemisia annua
<400> 1
   atggcccaaa aagctcccgg tgtcatcact tgtaaagccg cggtggtatg ggagtcaagt      60
   ggcccagtgg tgctcgagga gataagggtg gatccgccaa aagcatctga agttaggatc     120
   aagatgttgt gtgcaagcct ttgtcatact gacgtcttat gcaccaaagg atttcccatt     180
   cctttgtttc ctcgtattcc aggacatgaa ggtgttgggg tgattgaaag catagggaaa     240
   gacgcaaaag gtttgaaacc aggagacata gtgatgccac tctaccttgg tgaatgtgga     300
   caatgcttga actgcaaaac aggaaagacc aacctatgtc atgtttatcc accttcattt     360
   agtggcctca tgaacgatgg cacttctagg atgtccattg ccagaaccgg agaatctata     420
   tatcactttg caagttgttc aacatggaca gaatatgcag ttgctgactg caattatgtg     480
   ctcaagatca acccaaaaat ttcttaccct catgctagtt tcctctcatg tggcttcacc     540
   actggctttg gtgcgacgtg gagagaaacc caagtctcta agggctcctc agttgctgtt     600
   tttggcattg gtactgttgg tttaggggtg ataaaaggag cacagttgca aggagcatca     660
   aagataatag gagtggatgt aaaccagtat aaggcagcta aaggaaaagt  gtttggaatg     720
   accgatttta tcaacccaaa ggaccatcct gacaaatcag tttcagagtt ggtgaaggaa     780
   ctaactcatg gattaggcgt tgaccactgc tttgagtgca cgggtgtgcc atccttgttg     840
   aacgaagccc ttgaagcctc caaaattgga ataggtacag tggtaccaat aggagcgggt     900
   ggagaggcta gtgtggccat taacagcctg atactcttta gtggtcgcac tttgaagttc     960
   accgcttttg gtggcgtaag aacccaatct gatcttcctg tcataatcga taaatgcttg    1020
   aataaggaaa tacaacttga tgagcttttg acacatgaga tacatttgga caatatacaa    1080
   gaagcttttg agatattgaa gaagccagac tgtgttaaga ttctcatcaa attttaa      1137
<210> 2
   <211> 378
   <212> PRT
   <213> Artemisia annua
<400> 2
   Met Ala Gln Lys Ala Pro Gly Val Ile Thr Cys Lys Ala Ala Val Val
   1               5                   10                  15
   Trp Glu Ser Ser Gly Pro Val Val Leu Glu Glu Ile Arg Val Asp Pro
               20                  25                  30
   Pro Lys Ala Ser Glu Val Arg Ile Lys Met Leu Cys Ala Ser Leu Cys
           35                  40                  45
   His Thr Asp Val Leu Cys Thr Lys Gly Phe Pro Ile Pro Leu Phe Pro
       50                  55                  60
   Arg Ile Pro Gly His Glu Gly Val Gly Val Ile Glu Ser Ile Gly Lys
   65                  70                  75                  80
   Asp Ala Lys Gly Leu Lys Pro Gly Asp Ile Val Met Pro Leu Tyr Leu
                   85                  90                  95
   Gly Glu Cys Gly Gln Cys Leu Asn Cys Lys Thr Gly Lys Thr Asn Leu
               100                 105                 110
   Cys His Val Tyr Pro Pro Ser Phe Ser Gly Leu Met Asn Asp Gly Thr
           115                 120                 125
   Ser Arg Met Ser Ile Ala Arg Thr Gly Glu Ser Ile Tyr His Phe Ala
       130                 135                 140
   Ser Cys Ser Thr Trp Thr Glu Tyr Ala Val Ala Asp Cys Asn Tyr Val
   145                 150                 155                 160
   Leu Lys Ile Asn Pro Lys Ile Ser Tyr Pro His Ala Ser Phe Leu Ser
                   165                 170                 175
   Cys Gly Phe Thr Thr Gly Phe Gly Ala Thr Trp Arg Glu Thr Gln Val
               180                 185                 190
   Ser Lys Gly Ser Ser Val Ala Val Phe Gly Ile Gly Thr Val Gly Leu
           195                 200                 205
   Gly Val Ile Lys Gly Ala Gln Leu Gln Gly Ala Ser Lys Ile Ile Gly
       210                 215                 220
   Val Asp Val Asn Gln Tyr Lys Ala Ala Lys Gly Lys Val Phe Gly Met
   225                 230                 235                 240
   Thr Asp Phe Ile Asn Pro Lys Asp His Pro Asp Lys Ser Val Ser Glu
                   245                 250                 255
   Leu Val Lys Glu Leu Thr His Gly Leu Gly Val Asp His Cys Phe Glu
               260                 265                 270
   Cys Thr Gly Val Pro Ser Leu Leu Asn Glu Ala Leu Glu Ala Ser Lys
           275                 280                 285
   Ile Gly Ile Gly Thr Val Val Pro Ile Gly Ala Gly Gly Glu Ala Ser
       290                 295                 300
   Val Ala Ile Asn Ser Leu Ile Leu Phe Ser Gly Arg Thr Leu Lys Phe
   305                 310                 315                 320
   Thr Ala Phe Gly Gly Val Arg Thr Gln Ser Asp Leu Pro Val Ile Ile
                   325                 330                 335
   Asp Lys Cys Leu Asn Lys Glu Ile Gln Leu Asp Glu Leu Leu Thr His
               340                 345                 350
   Glu Ile His Leu Asp Asn Ile Gln Glu Ala Phe Glu Ile Leu Lys Lys
           355                 360                 365
   Pro Asp Cys Val Lys Ile Leu Ile Lys Phe
       370                 375

## Description

### Cross-reference to Related Applications

This application claims the benefit of United States Provisional Patent Application Serial No. 61/137,701 filed August 1, 2008, the entire contents of which is herein incorporated by reference.

### Field of the Invention

The present invention relates to production of plant-derived compounds of health and commercial interest. More particularly, the present invention relates to nucleotide sequences encoding an enzyme, to an enzyme encoded by a nucleotide sequence and to processes for producing artemisinic aldehyde and other precursors of artemisinin

### Background of the Invention

Plants, in general, contain a myriad of secondary metabolites often synthesized by unique biochemical processes operating only in exotic species. For plant-derived products such as drugs, the 1997 worldwide sales were US$ 10 billion (Rotheim 2002). In many cases the supply of the relevant plant material for these drugs is limited or variable. One approach to developing methods for producing these drugs is to apply the methods of biochemistry, molecular biology and genomics to elucidate the biosynthesis and relevant biosynthetic genes for compounds of value for human health.

With the realization that many of the enzymes involved in natural product biosynthesis represent variations within known classes of enzymes, expressed sequence tag (EST) analysis (combined with heterologous expression) provides a powerful means of identifying their corresponding genes (Cahoon et al. 1999; Gang et al. 2001; Lange et al. 2000; van de Loo, Turner, & Somerville 1995).

One area of interest is bioactive compounds of the tribe Anthemideae in the family Asteraceae (Compositae) (Torrell et al. 1999; Watson, Evans, & Boluarte 2000). Anthemideae (Asteraceae, subfamily Asteroideae) is a tribe of 109 genera which includes daisies, chrysanthemums, tarragon, chamomile, yarrow and sagebrushes (Watson, Evans, & Boluarte 2000). These plants are aromatic in nature resulting from high concentrations of mono- and sesqui- terpenes. Many of the species in this tribe are valued for the health benefits or insecticidal properties.

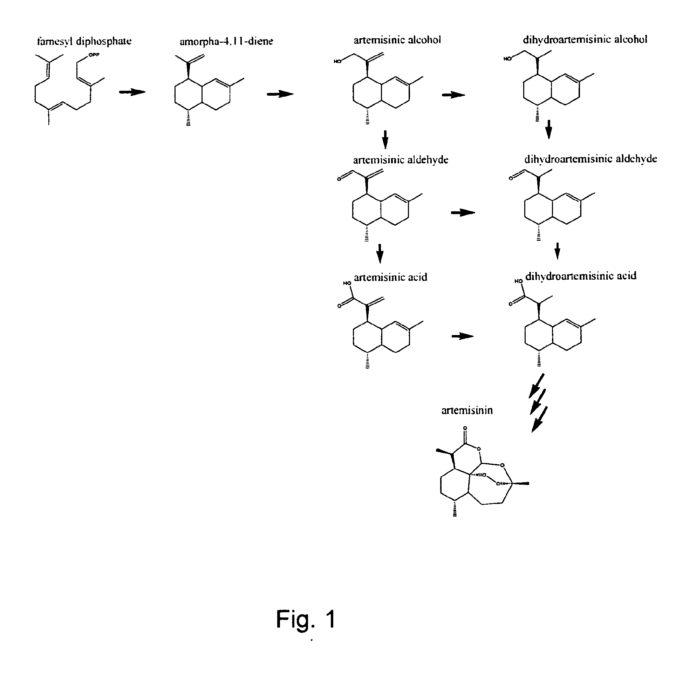

Of particular interest is artemisinin from *Artemisia annua* or sweet wormwood. In 1972, Chinese scientists reported the isolation of the sesquiterpene lactone containing an endoperoxide group (see Fig. 1) from *Artemisia* and called it qinghaosu (van Agtmael, Eggelte, & van Boxtel 1999). Prior to this sweet wormwood or qinghao had been used in traditional Chinese medicine for centuries. Artemisinin has become very important for the treatment of malaria in Southeast Asia and elsewhere, particularly for multi-drug-resistant falciparum forms of the disease (O'Neill 2005; Rathore et al. 2005; Robert, Coppel, & Meunier 2002; Wilairatana et al. 2002; Wu 2002). Since the discovery of artemisinin, a number of semi-synthetic derivatives have been developed for specific applications in malaria treatment.

Malaria remains a serious health problem which affects over 400 million people, especially in Africa and Southeast Asia, causing the deaths in excess of 2 million each year. Increasing resistance of the malaria parasite, *Plasmodium falciparum,* towards current antimalarial drugs is a cause for concern. The future value of antimalarial drugs based on the artemisinin structure is illustrated by the development by Bayer AG of Artemisone, an artemisinin derivative reported to be 10-30 fold more active than artesunate, for which clinical trials are currently under way.

Artemisinin is produced in relatively small amounts of 0.01 to 1.5% dry weight, making it and its derivatives relatively expensive (Gupta et al. 2002). Several studies describe the chemical synthesis of the sesquiterpene, but none are an economical alternative for isolation of artemisinin from the plant (Yadav, Babu, & Sabitha 2003). Therefore a higher concentration in the plant or production in an alternative host is desirable to make artemisinin available as economically as possible, especially for use in the Third World. Knowledge of the biosynthetic pathway and the genes involved should enable engineering of improved production of artemisinin. Alternatively, there is also the possibility of producing intermediates in the pathway to artemisinin which are of commercial value. For example, a compound 15 times more potent *in vitro* than artemisinin against *Plasmodium falciparum* has been synthesized from artemisinic alcohol (Jung, Lee, & Jung 2001).

There is evidence that artemisinin is localized to glandular trichomes on the surfaces of certain tissues of the plant (Duke et al. 1994; Duke & Paul 1993). The number and even existence of these trichomes and the amount of artemisinin varies widely among biotypes.

Typically, compounds discovered in plants and found to be useful are produced commercially by i) chemical synthesis, where possible and economical, ii) extraction of cultivated or wild plants, or iii) cell or tissue culture (this is rarely economical). In those cases in which chemical synthesis is not economical, it makes sense to learn as much as possible about the biosynthesis of a natural product, such that it can be produced most efficiently in plants or cell/tissue culture. In the case of artemisinin, chemical synthesis is not commercially feasible. Since the compound is produced in small quantities in *Artemisia,* the drugs derived from artemisinin are relatively expensive, particularly for the Third World countries in which they are used. While the antimalarial drugs, chloroquine and sulfadoxine-pyrimethamine, cost as little as 20 cents for an adult treatment, artemisinin-derived compounds, by contrast, can be 100 times as expensive. Chloroquine resistance is prevalent and sulfadoxine-pyrimethamine resistance is increasing. The World Health Organization recently added the artemisinin-derived drug, artemether to their Model List of Essential Medicines, which are recommended to be available at all times in adequate amounts and in the appropriate dosage forms, and at a price that individuals and the community can afford. Consequently, it would be useful to be able to supply artemisinin-derived drugs more economically.

There are numerous patents relating to artemisinin and artemisinin derived drugs. These cover drug synthesis and formulation, *Artemisia* cultivation (Kumar 2002) and tissue culture and artemisinin extraction (Elferaly 1990). In the past five years a reasonably clear picture of artemisinin biosynthesis has emerged as illustrated in Fig. 1 (Bertea et al. 2005). The identity of amorpha-4,11-diene as a biosynthetic intermediate was established, based on the presence of trace of amorpha-4,11-diene in *Artemisia* extracts and the cloning and expression of cDNAs representing amorpha-4,11-diene synthase, a terpene cyclase (Bouwmeester et al. 1999; Wallaart et al. 2001). A cytochrome P450 gene designated *cyp71av1* was recently cloned and characterized (Teoh et al. 2006). The *cyp71av1* gene encodes a hydroxylase that catalyzes the conversion of amorpha-4,11-diene to artemisinic alcohol. CYP71AV1 expressed in yeast is also capable of oxidizing artemisinic alcohol to artemisinic aldehyde and artemisinic aldehyde to artemisinic acid.

Commonly owned United States patent application 60/729,210 filed October 24, 2005, and now filed as a PCT patent application published May 3, 2007 under publication number WO 2007/048235, the disclosures of which are herein incorporated by reference, discloses a gene encoding amorpha-4,11-diene hydroxylase, which catalyzes the first committed steps in artemisinin biosynthesis (Fig. 1).

Commonly owned international patent application PCT/CA2007/000614 filed April 4, 2007 and published on October 11, 2007 under publication number WO 2007/112596, the disclosure of which is herein incorporated by reference, discloses AaSAD (Aldh1), an aldehyde dehydrogenase. The AaSAD disclosed in this publication oxidizes artemisinic aldehyde to artemisinic acid.

Chang et al. (Chang 2007) reported the use of modified CYP71AV1 in *E*. *coli* and the production of artemisinic alcohol, artemisinic aldehyde and artemisinic acid. Given that artemisinic acid is more desirable for the commercial production of artemisinin, there is a need for an enzyme capable of efficient oxidation of artemisinic alcohol to artemisinic aldehyde. The aldehyde could then be oxidized to the acid either by CYP71AV1 or *A*. *annua* Aldh1

### Summary of the Invention

The invention described herein addresses the production of artemisinin and artemisinin-related compounds, including precursors, of pharmaceutical and commercial interest.

There is provided an isolated nucleic acid molecule encoding an alcohol dehydrogenase capable of oxidizing artemisinic alcohol (*Artemisia annua* Adh1), the isolated nucleic acid molecule comprising a nucleotide sequence having at least 80% sequence identity to the nucleotide sequence as set forth in SEQ ID NO: 1.

There is provided a purified or partially purified alcohol dehydrogenase capable of oxidizing artemisinic alcohol comprising an amino acid sequence having at least 70% sequence identity to the amino acid sequence as set forth in SEQ ID NO: 2.

There is provided a purified or partially purified alcohol dehydrogenase capable of oxidizing artemisinic alcohol encoded by a nucleic acid molecule of the present invention.

There is provided a use of one or more purified or partially purified alcohol dehydrogenase capable of oxidizing artemisinic alcohol of the present invention in the production of artemisinic aldehyde.

There is provided a process for producing artemisinic aldehyde comprising expressing or overexpressing the isolated nucleic acid molecule of the present invention in a host cell.

There is provided a method of selecting or developing plants with altered artemisinic alcohol, artemisinic aldehyde, dihydroartemisinic aldehyde, dihydroartemisinic acid, artemisinic acid and/or artemisinin levels in a population of plants that naturally produces artemisinic alcohol, artemisinic aldehyde, dihydroartemisinic aldehyde, dihydroartemisinic acid, artemisinic acid and/or artemisinin comprising: detecting a target plant having altered levels of artemisinic alcohol, artemisinic aldehyde, dihydroartemisinic aldehyde, dihydroartemisinic acid, artemisinic acid and/or artemisinin compared to a control plant provided under similar conditions; isolating at least a portion of an alcohol dehydrogenase (Adh1) gene of the target plant and comparing the nucleotide sequence of said at least a portion to SEQ ID NO: 2 to detect a variation from SEQ ID NO: 2; detecting the variation in other plants; selectively breeding the plants with the variation to produce a population of plants having altered levels of artemisinic alcohol, artemisinic aldehyde, dihydroartemisinic aldehyde, dihydroartemisinic acid, artemisinic acid and/or artemisinin compared to a population of control plants produced under similar conditions.

There is provided a method of increasing artemisinic aldehyde, dihydroartemisinic aldehyde, dihydroartemisinic acid, artemisinic acid and/or artemisinin levels in a population of plants that naturally produces dihydroartemisinic aldehyde, dihydroartemisinic acid, artemisinic acid and/or artemisinin comprising: providing a population of mutated plants; detecting a target mutated plant within the population of mutated plants, the target mutated plant having an altered expression of an *Artemisia annua* Adh1 gene or altered activity of *A*. *annua* Adh1 enzyme compared to a control plant provided under similar conditions, said detecting comprising using primers developed from a nucleic acid molecule of the present invention to PCR amplify regions of the Adh1 gene from mutated plants in the population of mutated plants, identifying mismatches between the amplified regions and corresponding regions in the wild-type gene that lead to the altered expression or altered activity, and identifying the mutated plant that contains the mismatches; and, selectively breeding the target mutated plant to produce a population of plants having altered expression of the *A. annua Adh1* gene or altered alcohol dehydrogenase enzyme activity compared to a population of control plants produced under similar conditions.

The nucleotide sequence may have, at least 90%, at least 95% or at least 99% sequence identity to the nucleotide sequence as set forth in SEQ ID NO: 1. The amino acid sequence may have at least 80%, at least 90%, at least 95% or at least 99% sequence identity to the amino acid sequence as set forth in SEQ ID NO: 2.

The gene (nucleic acid molecule) of the present invention may be derived, for example cloned, from *Artemisia annua.* Obtaining other nucleic acid molecules of the present invention may be accomplished by well-known techniques in the art. Such techniques are disclosed in Sambrook et al. 2001 and Ausubel et al. eds. 2001. Generally, other nucleic acid molecules of the present invention may be obtained by: a) identifying the existence of a homologous gene by techniques such as, for example, hybridization of a target gene to the complement of SEQ ID NO: 1, genome or transcriptome (cDNA) sequencing, or database searching in nucleic acid sequence databases such as Genbank; b) cloning the homologous gene and creating a plasmid for *E*. *coli* or yeast expression as described in Sambrook et al. 2001 or Ausubel et al. eds. 2001; and, c) then testing the gene product for artemisinic alcohol oxidation Database searching may employ commonly used computer programs such as BLASTX, BLASTP, TBBLASTN and others.

Other nucleic acid molecules and proteins of the present invention may also be obtained by creating mutations of nucleic acid molecules and proteins already at hand. Such mutations may be accomplished by commonly known methods in the art as described in Sambrook et al. 2001 and Ausubel et al. eds. 2001.

Overexpression of one or more of the nucleic acid molecules or overproduction of the Adh1 enzyme may be done in *A*. *annua.* Expression of one or more of the nucleic acid molecules or expression of the alcohol dehydrogenase gene may be done in other hosts, for example plants, yeasts or bacteria. Overexpression or expression of one or more of the isolated nucleic acid molecules of the present invention may be done in combination with overexpression or expression of one or more other nucleic acid molecules involved in the biosynthesis of artemisinin, for example those encoding farnesyl diphosphate synthase, amorpha-4,11-diene synthase, amorpha-4,11-diene hydroxylase, double bond reductase, aldehyde dehydrogenase.

Part of the solution to the problem of producing artemisinin in an economical and timely fashion is the isolation and exploitation of genes involved in artemisinin biosynthesis. As in other examples of metabolic engineering, such genes can be used to enhance production by overexpression in the native plant (*A*. *annua*), a different plant, or in micro-organisms such as bacteria or yeast. An example of this is the expression of the amorphadiene synthase gene in *E*. *coli* to produce the artemisinin precursor amorphadiene (Martin et al. 2003) and the production of artemisinic acid in yeast (Ro et al. 2006). One important step in the pathway to artemisinin *per se*, is thought to be the oxidation of artemisinic alcohol to artemisinic aldehyde. Consequently, the gene involved in this step may be used to produce artemisinic aldehyde in a host, alone or in combination with one or more of farnesyl diphosphate synthase, amorpha-4,11-diene synthase, and amorpha-4,11-diene hydroxylase. The resulting artemisinic aldehyde could then be converted to artemisinic acid, dihydroartemisinic aldehyde or dihydroartemisinic acid with other known genes or enzymes.

The resulting artemisinic acid or dihydroartemisinic acid could then be chemically converted to artemisinin or related compounds of commercial value. For example, dihydroartemisinic acid is a presumed late precursor of artemisinin, and its transformation to artemisinin has been shown to occur spontaneously through photooxidation, requiring no enzyme intervention (Sy & Brown 2002; Wallaart et al. 1999). Consequently, using dihydroartemisinic acid

The nucleic acid molecule of the present invention may also be used in the development of DNA markers and in targeted mutagenesis techniques (e.g. TILLING (Targeting Induced Local Lesions IN Genomes)).

A genetic marker (DNA marker) is a segment of DNA with an identifiable physical location on a chromosome and associated with a particular gene or trait and whose inheritance can be followed. A marker can be a gene, or it can be some section of DNA with no known function. Because DNA segments that lie near each other on a chromosome tend to be inherited together, markers are often used as indirect ways of tracking the inheritance pattern of a gene that has not yet been identified, but whose approximate location in the genome is known. Thus, markers can assist breeders in developing populations of organism having a particular trait of interest. Gene-specific markers can be used to detect genetic variation among individuals which is more likely to affect phenotypes relating to the function of a specific gene. For example, variation in a gene-specific marker based on *A*. *annua Adh1*, rather than variation in an anonymous DNA marker, would be more likely linked to variation in content of artemisinin or related compounds, by virtue of its association with the relevant biosynthetic pathway. In one embodiment, a DNA marker for *Adh1* could be developed by sequencing the polymerase chain reaction amplified *Adh1* gene from a number of individual plants of *Artemisia annua.* Such sequencing would provide information about sequence polymorphisms within the gene. A range of methods available to those skilled in the art could be used to detect such polymorphisms, including cleaved amplified polymorphic sequences (CAPs) (Konieczny & Ausubel 1993).

The presence of such gene-specific polymorphisms could be correlated with levels of artemisinin or related compounds and used in a breeding program to select and/or develop lines of *Artemisia annua* with enhanced levels of artemisinin or related compounds. That is, the variation in genetic structure may be detected in other plants, and the plants with the variation selectively bred to produce a population of plants having increased levels of dihydroartemisinic aldehyde, dihydroartemisinic acid artemisinic acid and/or artemisinin compared to a population of control plants produced under similar conditions. Genetic markers are discussed in more detail in Bagge et al. 2007, Pfaff et al. 2003, Sandal et al. 2002 and Stone et al. 2002.

TILLING (Bagge, Xia, & Lubberstedt 2007; Comai & Henikoff 2006; Henikoff, Till, & Comai 2004; Slade & Knauf 2005) involves treating seeds or individual cells with a mutagen to cause point mutations that are then discovered in genes of interest using a sensitive method for single-nucleotide mutation detection. Detection of desired mutations (e.g. mutations resulting in a change in expression of the gene product of interest) may be accomplished, for example, by PCR methods. For example, oligonucleotide primers derived from the gene (nucleic acid molecule) of interest, such as the nucleic acid molecules of the present invention, may be prepared and PCR may be used to amplify regions of the gene of interest from plants in the mutagenized population. Amplified mutant genes may be annealed to wild-type genes to find mismatches between the mutant genes and wild-type genes. Detected differences may be traced back to the plants which had the mutant gene thereby revealing which mutagenized plants will have the desired expression. These plants may then be selectively bred to produce a population having the desired expression.

Further features of the invention will be described or will become apparent in the course of the following detailed description.

### Brief Description of the Drawings

In order that the invention may be more clearly understood, embodiments thereof will now be described in detail by way of example, with reference to the accompanying drawings, in which:
Fig. 1 depicts the proposed biosynthetic pathway for artemisinin biosynthesis.

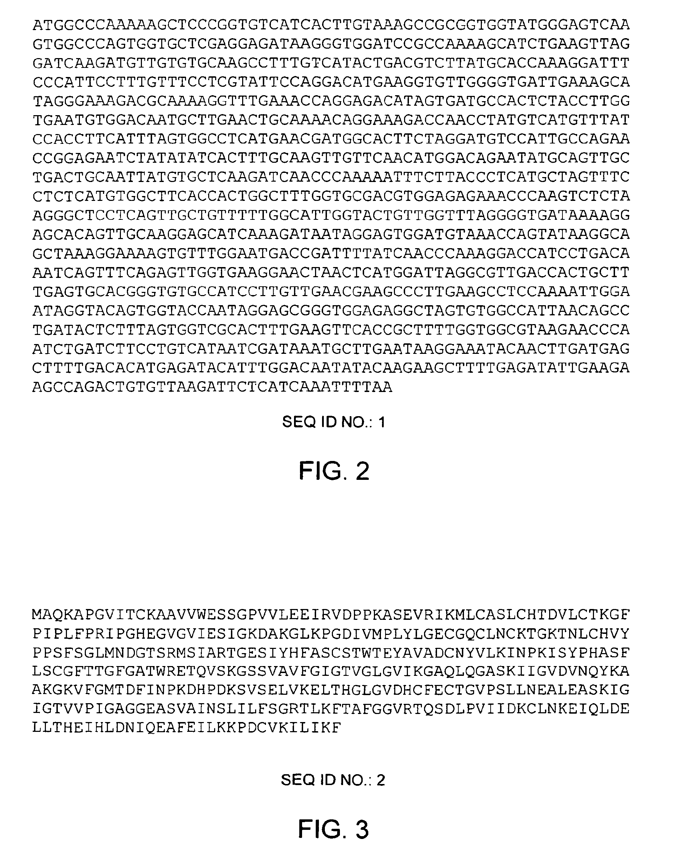

Fig. 2 depicts the nucleotide sequence (SEQ ID NO: 1) of the *Artemisia annua Adh1 cDNA* encoding alcohol dehydrogenase.
Fig. 3 depicts the predicted amino acid sequence (SEQ ID NO: 2) of the protein encoded by *Artemisia annua Adh1.*
Fig. 4 depicts a GC/MS analysis of alcohol dehydrogenase assays using artemisinic alcohol and dihydroartemisinic alcohol as substrates.

### Description of Preferred Embodiments

### Materials and Methods:

### Artemisinic aldehyde

Artemisinic acid was isolated from dichloromethane extracts of *A*. *annua* flower buds and leaves (Teoh, Polichuk, Reed, Nowak, & Covello 2006) and was used to synthesize artemisinic aldehyde according to the method described by Chang et al. 2000, the disclosure of which is incorporated herein by reference.

### Dihydroartemisinic aldehyde

Dihydroartemisinic aldehyde was synthesized from the isolated dihydroartemisinic acid (see above). The acid was converted to methyl dihydroartemisinate with excess diazomethane in diethyl ether at 0°C for 5 minutes. The ether and diazomethane were removed under a stream of nitrogen and the methyl ester was reduced to (*11R*)-dihydroartemisinic alcohol with excess 1.5 M diisobutyl aluminum hydride in toluene at room temperature for 10 min under nitrogen. With subsequent extraction, oxidation to the aldehyde with pyridinium chlorochromate (Corey & Suggs 1975) and purification by HPLC the (*11R*)-dihydroartemisinic aldehyde was produced at an overall yield of 48% with >90% purity according to GC analysis.

### Plant materials

*Artemisia annua L.* seeds were obtained from Elixir Farm Botanicals, Brixey, Missouri, USA and from Pedro Melillo de Magalhaes, State University of Campinas, Brazil (line 2/39). Seeds were germinated and grown in soil in a controlled environment chamber with 16 hour/25°C days and 8 hour/20°C nights. Plants that had reached the height of approximately 1.2 m (about 3 months) were transferred to flowering chamber with 12 hour/25 C days and 12 hour/20 C nights for the Elixir line and 8 hour/25°C days and 16 hour/20°C nights for line 2/39. Flower buds that developed after 19-21 days in the flowering chamber were harvested for total RNA isolation.

### cDNA library construction and expressed sequence tag (EST) analysis

Total RNA was extracted and isolated from glandular trichomes and flower buds using a modified method described by Logeman, et al. 1987. cDNA synthesis from 1.5 micrograms of total RNA and construction of the trichome and flower bud cDNA library were carried out with Creators^{™} SMART^{™} cDNA Library Construction Kit (Clontech). A total of 6,239 clones and 2,208 clones for trichome and flower bud libraries, respectively were randomly picked and their DNA sequences determined. Sequencing was performed on an AB13700 DNA sequencer using BigDye Terminator Cycle Sequencing Kit (Applied Biosystems) and the M13 reverse primer. DNA sequence traces were interpreted and vector and low quality sequences were eliminated using PHRED (Ewing et al. 1998) and LUCY (Chou & Holmes 2001). Clustering of the resulting EST dataset was done using STACKPACK (Miller et al. 1999) and sequence similarity was identified by BLAST (Altschul et al. 1990).

### Isolation of full-length Artemisia annua Adh1 cDNA

The gene corresponding to an *Artemisia annua* cDNA clone was identified by sequence similarity searches as showing similarity to other nucleotide sequences encoding alcohol dehydrogenases was designated *Artemisia annua Adh1.* The nucleotide sequence of the open reading frame of Adh1 is given by SEQ ID NO: 1.

### Expression of Adh1

The Gateway vector pDEST17 had the Adh1 gene inserted and was subsequently transformed into a competent expression *E. coli* strain Rosetta (DE3). These cells were grown overnight in 3 ml of LB broth amended with 100 µg/ml ampicillin. An aliquot of 1 ml overnight culture was sub cultured to 100 ml of fresh LB broth also amended with 100 µg/ml of ampicillin. The culture was incubated at 37°C until the optical density reached 0.8 at 600 nm. A final concentration of 1 mM IPTG was then added to the culture and the flask was incubated at 30°C for another 4 hours. Cells were harvested by centrifugation at 10,000 rcf for 10 minutes. Pelleted cells were resuspended in 20 mM sodium phosphate pH 7.4 and 500 mM NaCl. Cell walls were disrupted by sonication on ice at intervals of 0.7 seconds for 1 minute, followed by a 1 minute cool down this was repeated three times. The sonicated cells were centrifuged at 10,000 rcf for 10 minutes; the supernatant which contained the soluble ADH1 was concentrated and desalted by spin dialysis (using Amicon Ultra-15 devices; Millipore, MA), and stored in a new tube at 4°C. This method of recombination and expression was also used for a control plasmid pDEST17-gus.

### Specific ADH1 Activity

Enzyme assay was prepared to determine the specific activity of ADH1. The assay contained a final concentration of 1 mM nicotinamide adenine dinucleotide (NAD⁺), 0.1 µg/µl artemisinic alcohol or dihydroartemisinic alcohol (AAOH or DHAAOH) and approximately 90 µg of crude protein. The assays were incubated at 30°C for 30 minutes at 500 rpm, followed by an ethyl acetate extraction. The upper organic phase was transferred to a GC vial and the sample was run on an Agilent 6890N - Network GC System coupled to an Agilent 5973 Network Mass Selective Detector. The column present in the GC was an Innowax column (30 m, I.D. 0.250 mm, Film 0.25 µm), with a 50:1 split injector, the temperature gradient began at 125°C and increased at 5°C/minute to a maximum temperature of 300°C.

### Results:

### Specific ADH1 Activity

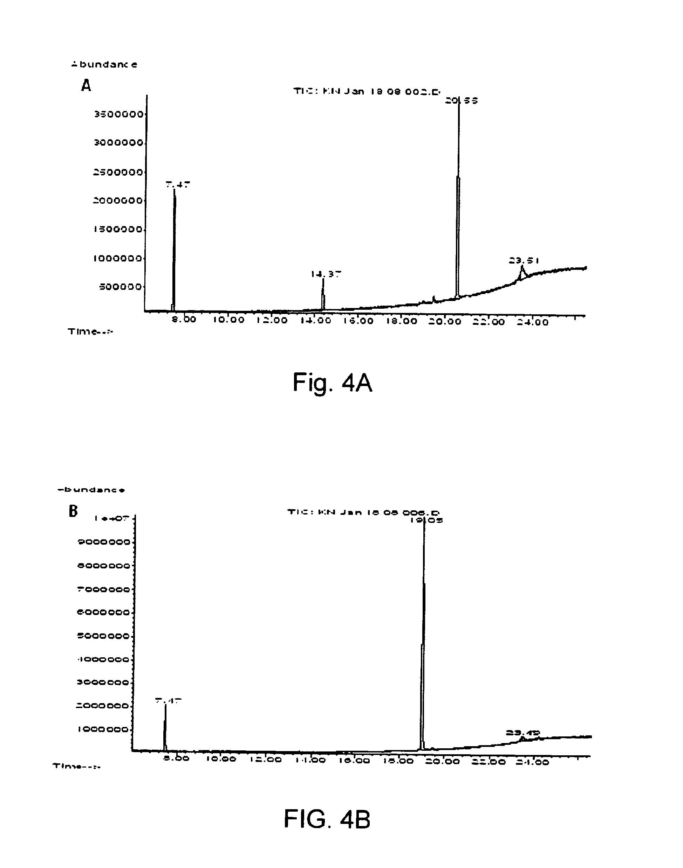

Enzyme assays were prepared to determine whether ADH1 acted specifically on artemisinic alcohol or if there was also activity observed with dihydroartemisinic alcohol as a substrate. These substrates were tested with cells that were expressing the ADH1 gene as well as a control gus gene. It was observed that ADH1 acts on AAOH and not DHAAOH (Fig. 4). Fig. 4A shows GC/MS analysis of crude Adh1 incubated with artemisinic alcohol (retention time = 20.55 min), and the presence of product artemisinic aldehyde (14.37 min). Peaks were confirmed by mass spectrometry. Fig. 4B shows GC/MS analysis of crude Adh1 incubated with dihydroartemisinic alcohol (19.05 min), and no observed product. No product was observed with extracts of *E. coli* transformed with the control plasmid pDEST17-gus.

### Sequences:

SEQ ID NO: 1 *- Artemisia annua* (alcohol dehydrogenase gene) (1137 bp)
SEQ ID NO: 2 - *Artemisia annua* (alcohol dehydrogenase) (378 aa)

### References:

Altschul, S. F., Gish, W., Miller, W., Myers, E. W., & Lipman, D. J. (1990), "Basic local alignment search tool", Journal of molecular Biology, vol. 215, pp. 403-410.
Ausubel et al. eds. (2001), Current Protocols in Molecular Biology, (John Wiley & Sons, Somerset, N.J.).
Bagge, M., Xia, X., & Lubberstedt, T. (2007); "Functional markers in wheat", CurrOpin.Plant Biol., vol. 10, no. 2, pp. 211-216.
Bertea, C. M., Freije, J. R., van der Woude H., Verstappen, F. W., Perk, L., Marquez, V., de Kraker, J. W., Posthumus, M. A., Jansen, B. J., de Groot, A., Franssen, M. C., & Bouwmeester, H. J. (2005), 'Identification of intermediates and enzymes involved in the early steps of artemisinin biosynthesis in Artemisia annua", Planta Med., vol. 71, no. 1, pp. 40-47.
Bouwmeester, H. J., Wallaart, T. E., Janssen, M. H., van Loo, B., Jansen, B. J., Posthumus, M. A., Schmidt, C. O., de Kraker, J. W., Konig, W. A., & Franssen, M. C. (1999), "Amorpha-4,11-diene synthase catalyses the first probable step in artemisinin biosynthesis", Phytochemistry, vol. 52, no. 5, pp. 843-854.
Cahoon, E. B., Carlson, T. J., Ripp, K. G., Schweiger, B. J., Cook, G. A., Hall, S. E., & Kinney, A. J. (1999), "Biosynthetic origin of conjugated double bonds: production of fatty acid components of high-value drying oils in transgenic soybean embryos", Proceedings of the National Academy of Sciences (U.S.A.), vol. 96, pp. 12935-12940.
Chang, Y. J., Song, S. H., Park, S. H., & Kim, S. U. (2000), "Amorpha-4,11-diene synthase of Artemisia annua: cDNA isolation and bacterial expression of a terpene synthase involved in artemisinin biosynthesis", Archives of Biochemistry and Biophysics, vol. 383, no. 2, pp. 178-184.
Chang, M.C.Y., Eachus, R.A., Trieu, W., Ro, D.-K. and Keasling, J.D. (2007), "Engineering Escherichia coli for production of functionalized terpenoids using plant P450s", Nature Chem. Biol., vol. 3, pp. 274-277.
Chou, H.-H. & Holmes, M. H. (2001), "DNA sequence quality trimming and vector removal", Bioinformatics, vol. 17, pp. 1093-1104.
Comai, L. & Henikoff, S. (2006), 'TILLING: practical single-nucleotide mutation discovery", The Plant Journal, vol. 45, no. 4, pp. 684-694.
Corey, E. J. & Suggs, J. W. (1975), "Pyridinium chlorochromate: An efficient reagent for oxidation of primary and secondary alcohols to carbonyl compounds", Tetrahedron Letters, vol. 31, pp. 2647-2650.
Duke, M. V., Paul, R. N., Elsohly, H. N., Sturtz, G., & Duke, S. O. (1994), "Localization of artemisinin and artemisitene in foliar tissues of glanded and glandless biotypes of Artemisia annua", Int.J.Plant Sci., vol. 155, pp. 365-372.
Duke, S. O. & Paul, R. N. (1993), "Development and fine structure of the glandular trichomes of Artemisia annua L.", Int.J.Plant Sci., vol. 154, pp. 107-118.
Elferaly, F. S. (1990), Method for the isolation of artemisinin from *Artemisia annua,* U.S. patent 4,952,603.
Ewing, B., Hillier, L., Wendi, M. C., & Green, P. (1998), "Base-calling of automated sequencer traces using phred I. Accuracy assessment", Genome Res., vol. 8, pp. 175-185.
Gang, D. R., Wang, J., Dudareva, N., Nam, K. H., Simon, J. E., Lewinsohn, E., & Pichersky, E. (2001), "An investigation of the storage and biosynthesis of phenylpropenes in sweet basil", Plant Physiol, vol. 125, no. 2, pp. 539-555.
Gupta, S. K., Singh, P., Bajpai, P., Ram, G., Singh, D., Gupta, M. M., Jain, D. C., Khanuja, S. P., & Kumar, S. (2002), "Morphogenetic variation for artemisinin and volatile oil in Artemisia annua", Ind Crops Products, vol. 16, pp. 217-224.
Henikoff, S., Till, B. J., & Comai, L. (2004), 'TILLING. Traditional mutagenesis meets functional genomics", Plant Physiol, vol. 135, no. 2, pp. 630-636.
Jung, M., Lee, K., & Jung, H. (2001), "First synthesis of (+)-deoxyartemisitene and its novel C-11 derivatives", Tetrahedron Lett, vol. 42, pp. 3997-4000.
Konieczny, A. & Ausubel, F. M. (1993), "A procedure for mapping Arabidopsis mutations using co-dominant ecotype-specific PCR-based markers", The Plant Journal, vol. 4, no. 2, pp. 403-410.
Kumar, S. (2002), Method for maximization of artemisinin production by the plant *Artemisia annua,* U.S. patent 6,393,763.
Lange, B. M., Wildung, M. R., Stauber, E. J., Sanchez, C., Pouchnik, D., & Croteau, R. (2000), "Probing essential oil biosynthesis and secretion by functional evaluation of expressed sequence tags from mint glandular trichomes", Proc.Natl.Acad. Sci. U.S.A, vol. 97, no. 6, pp. 2934-2939.
Logeman, J., Schell, J., & Willmitzer, L. (1987), "Improved Method for the Isolation of RNA from Plant Tissues", Analytical Biochemistry, vol. 163, pp. 16-20.
Martin, V. J., Pitera, D. J., Withers, S. T., Newman, J. D., & Keasling, J. D. (2003), "Engineering a mevalonate pathway in Escherichia coli for production of terpenoids", Nat. Biotechnol., vol. 21, no. 7, pp. 796-802.
Miller, R. T., Christoffels, A. G., Gopalakrishnan, C., Burke, J., Ptitsyn, A. A., Broveak, T. R., & Hide, W. A. (1999), "A comprehensive approach to clustering of expressed human gene sequence: the sequence tag alignment and consensus knowledge base", Genome Res., vol. 9, no. 11, pp. 1143-1155.
O'Neill, P. M. (2005), "The therapeutic potential of semi-synthetic artemisinin and synthetic endoperoxide antimalarial agents", Expert Opin.Investig.Drugs, vol. 14, no. 9, pp. 1117-1128.
Pfaff, T. & Kahl, G. (2003), "Mapping of gene-specific markers on the genetic map of chickpea (Cicer arietinum L.)", Mol. Genet. Genomics, vol. 269, no. 2, pp. 243-251.
Rathore, D., McCutchan, T. F., Sullivan, M., & Kumar, S. (2005), "Antimalarial drugs: current status and new developments", Expert Opin.Investig. Drugs, vol. 14, no. 7, pp. 871-883.
Ro, D. K., Paradise, E. M., Ouellet, M., Fisher, K. J., Newman, K. L., Ndungu, J. M., Ho, K. A., Eachus, R. A., Ham, T. S., Kirby, J., Chang, M. C., Withers, S. T., Shiba, Y., Sarpong, R., & Keasling, J. D. (2006), "Production of the antimalarial drug precursor artemisinic acid in engineered yeast", Nature, vol. 440, no. 7086, pp. 940-943.
Robert, A., Coppel, Y., & Meunier, B. (2002), "Alkylation of heme by the antimalarial drug artemisinin", Chem.Commun.(Camb.) no. 5, pp. 414-415.
Rotheim, P. (2002), Plant-Derived Drugs: Products, Technologies and Applications, Business Communications Co., Norwalk, B-121.
Sambrook, J. et al. (2001), Molecular Cloning: A Laboratory Manual (Third Edition) (Cold Spring Harbor Laboratory Press, Cold Spring Harbor, N.Y.).
Sandal, N., Krusell, L., Radutoiu, S., Olbryt, M., Pedrosa, A., Stracke, S., Sato, S., Kato, T., Tabata, S., Pamiske, M., Bachmair, A., Ketelsen, T., & Stougaard, J. (2002),"A genetic linkage map of the model legume Lotus japonicus and strategies for fast mapping of new loci", Genetics, vol. 161, no. 4, pp. 1673-1683.
Slade, A. J. & Knauf, V. C. (2005), "TILLING moves beyond functional genomics into crop improvement", Transgenic Res., vol. 14, no. 2, pp. 109-115.
Stone, R. T., Grosse, W. M., Casas, E., Smith, T. P., Keele, J. W., & Bennett, G. L. (2002), "Use of bovine EST data and human genomic sequences to map 100 gene-specific bovine markers", Mammalian Genome, vol. 13, no. 4, pp. 211-215.
Sy, L. K. & Brown, G. D. (2002), "The role of the 12-carboxylic acid group in the spontaneous autoxidation of dihydroartemisinic acid", Tetrahedron, vol. 58, pp. 909-923.
Teoh, K. H., Polichuk, D. R., Reed, D. W., Nowak, G., & Covello, P. S. (2006), "Artemisia annua L. (Asteraceae) trichome-specific cDNAs reveal CYP71AV1, a cytochrome P450 with a key role in the biosynthesis of the antimalarial sesquiterpene lactone artemisinin", FEBS Letters, vol. 580, no. 5, pp. 1411-1416.
Teoh, K. H., Reed, D. W., Polichuk, D. R., & Covello, P. S. (2007), World Patent publication WO 2007/112596.
Torrell, M., Garcia-Jacas, N., Susanna, A., & Valles, J. (1999), "Phylogeny in Artemisia (Asteraceae, Anthemideae) inferred from nuclear ribosomal DNA (ITS) sequences", Taxon, vol. 48, p. 721.
Trotter, E. W., Collinson, E. J., Dawes, I. W., & Grant, C. M. (2006), "Old yellow enzymes protect against acrolein toxicity in the yeast Saccharomyces cerevisiae", Applied and Environmental Microbiology, vol. 72, no. 7, pp. 4885-4892.
van Agtmael, M. A., Eggelte, T. A., & van Boxtel, C. J. (1999), "Artemisinin drugs in the treatment of malaria: from medicinal herb to registered medication", Trends Pharmacol. Sci., vol. 20, no. 5, pp. 199-205.
van de Loo, F. J., Turner, S., & Somerville, C. (1995), "Expressed sequence tags from developing castor seeds", Plant Physiology, vol. 108, pp. 1141-1150.
Wallaart, T. E., Bouwmeester, H. J., Hille, J., Poppinga, L., & Maijers, N. C. (2001), "Amorpha-4,11-diene synthase: cloning and functional expression of a key enzyme in the biosynthetic pathway of the novel antimalarial drug artemisinin", Planta, vol. 212, no. 3, pp. 460-465.
Wallaart, T. E., van Uden, W., Lubberink, H. G., Woerdenbag, H. J., Pras, N., & Quax, W. J. (1999), "Isolation and identification of dihydroartemisinic acid from Artemisia annua and its possible role in the biosynthesis of artemisinin", J.Nat.Prod., vol. 62, no. 3, pp. 430-433.
Watson, L. E., Evans, T. M., & Boluarte, T. (2000), "Molecular phylogeny and biogeography of tribe Anthemideae (Asteraceae), based on chloroplast ndhF", Mo/. Phylogen. Evol., vol. 15, pp. 59-69.
Wilairatana, P., Krudsood, S., Treeprasertsuk, S., Chalermrut, K., & Looareesuwan, S. (2002), "The future outlook of antimalarial drugs and recent work on the treatment of malaria", Arch.Med.Res., vol. 33, no. 4, pp. 416-421.
Wu, Y. (2002), "How might qinghaosu (artemisinin) and related compounds kill the intraerythrocytic malaria parasite? A chemist's view", Acc.Chem.Res., vol. 35, no. 5, pp. 255-259.
Yadav, J. S., Babu, R. S., & Sabitha, G. (2003), "Stereoselective total synthesis of (+)-artemisinin", Tetrahedron Lett, vol. 44, pp. 387-389.

## Claims

1. Isolated nucleic acid molecule comprising a nucleotide sequence having at least 80% nucleotide sequence identity to the nucleotide sequence as set forth in SEQ ID NO: 1, and encoding an alcohol dehydrogenase.

2. Isolated nucleic acid molecule comprising a nucleotide sequence encoding an alcohol dehydrogenase having an amino acid sequence with at least 70% amino acid sequence identity to SEQ ID NO: 2.

3. The nucleic acid molecule according to claim 2, wherein the amino acid sequence identity is at least 90%.

4. The nucleic acid molecule according to claim 2, wherein the amino acid sequence identity is at least 95%.

5. The isolated nucleic acid molecule according to any one of claims 1 to 4 having a nucleotide sequence comprising SEQ ID NO: 1.

6. The isolated nucleic acid molecule of any one of claims 1 to 5 derived from *Artemisia annua.*

7. Purified or partially purified alcohol dehydrogenase having an amino acid sequence with at least 70% amino acid sequence identity to SEQ ID NO: 2.

8. The dehydrogenase according to claim 7, wherein the amino acid sequence identity is at least 90%.

9. The dehydrogenase according to claim 7, wherein the amino acid sequence identity is at least 95%.

10. The dehydrogenase according to claim 7 having an amino acid sequence comprising SEO ID NO: 2.

11. Process for producing artemisinic aldehyde comprising expressing or overexpressing one or more isolated nucleic acid molecules as defined in any one of claims 1 to 6 in a host cell.

12. The process according to claim 11, further comprising expressing or overexpressing in the host cell one or more nucleic acid molecules encoding farnesyl diphosphate synthase, amorpha-4,11-diene synthase, amorpha-4,11-diene hydroxylase, double bond reductase, aldehyde dehydrogenase, or any combination thereof.

13. The process according to any one of claims 11 to 12, wherein the host cell is a plant cell.

14. The process according to any one of claims 11 to 12, wherein the host cell is a yeast cell or a bacterial cell.

## Patentansprüche

1. Isoliertes Nukleinsäuremolekül mit einer Nukleotidsequenz, die mindestens 80 % Nukleotidsequenzidentität mit SEQ ID NR : 1 aufweist und eine Alkoholdehydrogenase kodiert.

2. Isoliertes Nukleinsäuremolekül, aufweisend eine Nukleotidsequenz, die eine Alkoholdehydrogenase kodiert, deren Aminosäuresequenz zu mindestens 70 % mit SEQ ID NR : 2 identisch ist.

3. Nukleinsäuremolekül nach Anspruch 2, wobei die Aminosäuresequenzidentität mindestens 90 % beträgt.

4. Nukleinsäuremolekül nach Anspruch 2, wobei die Aminosäuresequenzidentität mindestens 95 % beträgt.

5. Isoliertes Nukleinsäuremolekül nach einem der Ansprüche 1 bis 4, dessen Nukleotidsequenz SEQ ID NR : 1 umfasst.

6. Isoliertes Nukleinsäuremolekül nach einem der Ansprüche 1 bis 5, das aus *Artemisia annua* stammt.

7. Gereinigte oder teilweise gereinigte Alkoholdehydrogenase, deren Aminosäuresequenz zu mindestens 70 % mit SEQ ID NR : 2 identisch ist.

8. Dehydrogenase nach Anspruch 7, wobei die Aminosäuresequenzidentität mindestens 90 % beträgt.

9. Dehydrogenase nach Anspruch 7, wobei die Aminosäuresequenzidentität mindestens 95 % beträgt.

10. Dehydrogenase nach Anspruch 7, deren Aminosäuresequenz SEQ ID NR : 2 umfasst.

11. Verfahren zum Herstellen eines artemisinischen Aldehyds, aufweisend das Exprimieren oder Überexprimieren eines oder mehrerer der isolierten Nukleinsäuremoleküle, gemäß einem der Ansprüche 1 bis 6, in einer Wirtszelle.

12. Verfahren nach Anspruch 11, zusätzlich umfassend das Exprimieren oder Überexprimieren eines oder mehrerer Nukleinsäuremoleküle, die Farnesyldiphosphatsynthase, Amorpha-4,11-diensynthase, Amorpha-4,11-dienhydroxylase, Doppelbindungsreduktase, Aldehyddehydrogenase kodieren, oder eine beliebige Kombination davon, in der Wirtszelle.

13. Verfahren nach einem der Ansprüche 11 bis 12, wobei es sich bei der Wirtszelle um eine Pflanzenzelle handelt.

14. Verfahren nach einem der Ansprüche 11 bis 12, wobei es sich bei der Wirtszelle um eine Hefezelle oder eine Bakterienzelle handelt.

## Revendications

1. Molécule d'acide nucléique isolée comprenant une séquence de nucléotides présentant une identité de séquence de nucléotides d'au moins 80 % avec la séquence de nucléotides telle qu'exposée dans SEQ ID NO : 1, et codant pour une alcool déshydrogénase.

2. Molécule d'acide nucléique isolée comprenant une séquence de nucléotides codant pour une alcool déshydrogénase ayant une séquence d'acides aminés présentant une identité de séquence d'acides aminés d'au moins 70 % avec SEQ ID NO : 2.

3. Molécule d'acide nucléique selon la revendication 2, dans laquelle l'identité de séquence d'acides aminés est d'au moins 90 %.

4. Molécule d'acide nucléique selon la revendication 2, dans laquelle l'identité de séquence d'acides aminés est d'au moins 95 %.

5. Molécule d'acide nucléique isolée selon l'une quelconque des revendications 1 à 4, ayant une séquence de nucléotides comprenant SEQ ID NO : 1.

6. Molécule d'acide nucléique isolée selon l'une quelconque des revendications 1 à 5, dérivée d'*Artemisia annua.*

7. Alcool déshydrogénase purifiée ou partiellement purifiée, ayant une séquence d'acides aminés présentant une identité de séquence d'acides aminés d'au moins 70 % avec SEQ ID NO : 2.

8. Déshydrogénase selon la revendication 7, dans laquelle l'identité de séquence d'acides aminés est d'au moins 90 %.

9. Déshydrogénase selon la revendication 7, dans laquelle l'identité de séquence d'acides aminés est d'au moins 95 %.

10. Déshydrogénase selon la revendication 7, ayant une séquence d'acides aminés comprenant SEQ ID NO : 2.

11. Procédé de production d'aldéhyde artémisinique comprenant l'expression ou la surexpression d'une ou de plusieurs molécules d'acide nucléique isolées telles que définies dans l'une quelconque des revendications 1 à 6 dans une cellule hôte.

12. Procédé selon la revendication 11, comprenant en outre l'expression ou la surexpression dans la cellule hôte d'une ou de plusieurs molécules d'acide nucléique codant pour la farnésyl diphosphate synthase, l'amorpha-4,11-diène synthase, l'amorpha-4,11-diène hydroxylase, la réductase à double liaison, l'aldéhyde déshydrogénase, ou toute combinaison de celles-ci.

13. Procédé selon l'une quelconque des revendications 11 à 12, dans lequel la cellule hôte est une cellule végétale.

14. Procédé selon l'une quelconque des revendications 11 à 12, dans lequel la cellule hôte est une cellule de levure ou une cellule bactérienne.
